# EUROPEAN PATENT APPLICATION

(11) **EP 4 840 419 A1**
(43) Date of publication of application: **18.11.2026**
(21) Application number: 24921665.6
(22) Date of filing: 27.11.2024
(51) Int. Cl.: A61B 34/30, A61B 34/00, A61B 17/00

(54) **OPERATION HANDLE AND IN-POSITION DETECTION METHOD FOR OPERATION HANDLE**

(30) Priority: 30.01.2024 CN 202410131830
(71) Applicant: Ronovo (Shanghai) Medical Science and Technology Ltd., Shanghai 200233 (CN)
(72) Inventor: YANG, Wen, Shanghai 200233 (CN)
(74) Representative: Cabinet Beaumont
(86) International application number: PCT/CN2024/134834
(87) International publication number: WO 2025/161643

(57) **Abstract**

Provided are an operating handle and an in-position detection method for an operating handle. The operating handle includes a handle body (1), an in-position detection module, and a control module. The in-position detection module includes an electrode disposed on the surface of the handle body (1); and a detection unit, which is electrically connected to the electrode, is communicatively connected to the control module, and is configured to acquire the voltage of the electrode and determine, based on a change in the voltage, whether a finger is in position. In this manner, an accurate and reliable in-position detection solution can be provided by disposing the electrode on the surface of the handle body (1) and determining, by the detection unit based on the change in the voltage of the electrode, whether the finger is in position. This detection solution, using the change in the voltage as the basis for determination, has a relatively simple circuit, is less susceptible to interference, and exhibits high reliability, thereby avoiding the problem that in-position detection is prone to failure when the finger is relatively moist.

## Description

This application claims priority to Chinese Patent Application No. 202410131830.1 filed on Jan. 30, 2024, the disclosure of which is incorporated herein by reference in its entirety.

### TECHNICAL FIELD

The present application relates to the field of medical devices and, in particular, to an operating handle and an in-position detection method for an operating handle.

### BACKGROUND

In a surgical robot system, an operating handle of a surgeon console is used to control the movement of an instrument on a patient-side platform. Since the instrument is located inside a patient's body, the movement of the instrument must be strictly controlled by a surgeon. It is necessary to prevent the operating handle from moving freely after the fingers of the surgeon slip off the operating handle and from causing an inadvertent contact that could harm the patient. Therefore, the reliability of in-position detection for determining whether the operating handle is being held by the fingers of the surgeon is extremely important. At present, the following solutions are commonly used to perform the in-position detection.
(1) The first method is to perform indirect measurement through a handle angle sensor. During the operation, the surgeon needs to pinch the operating handle first. The angle sensor obtains information indicating that the hand of the surgeon is in position, thereby fulfilling the in-position detection function. However, this method cannot distinguish between two operating conditions: the hand of the surgeon being withdrawn from the operating handle and the surgeon controlling the operating handle to be in a non-pinch state. Therefore, the instrument on the operating arm cannot be locked promptly when the surgeon's hand is withdrawn, which introduces risks during surgery.
(2) The second conventional method is to place infrared distance sensors on the operating handle and near the operating handle. When the finger is in position, the hand blocks the sensors, thereby changing the distance values read by the sensors. A preset threshold is used to determine whether the hand is in position. The disadvantage of this method is that misjudgment can easily occur. When the operating handle is moved to some positions, other objects may block the sensors, causing the sensors to incorrectly determine that the hand is in position.
(3) The third method is to capture a video through a camera and extract hand states and features based on image recognition to determine whether the hand is in position. The drawback of this solution is that the surgeon's hand may be occluded at some positions during surgery. As a result, extracting the hand features from video information can easily lead to misjudgment.

The detection accuracy of the second method and the third method can be improved by increasing the number of sensors. However, the computational load and cost can also be exponentially increased, resulting in a more complex structure for the operating handle.

### SUMMARY

The technical problem to be solved by the present application is to overcome the defects in the existing art that the in-position detection function of the operating handle has a complex structure and low detection accuracy, and the present application provides an operating handle and an in-position detection method for an operating handle.

The present application solves the above technical problem with the following technical solutions.

An operating handle is provided and includes a handle body, an in-position detection module, and a control module, and the in-position detection module includes an electrode and a detection unit.

The electrode is disposed on a surface of the handle body.

The detection unit is electrically connected to the electrode and is communicatively connected to the control module, and the detection unit is configured to acquire a voltage of the electrode and determine, based on a change in the voltage, whether a finger is in position.

According to the preceding operating handle, an accurate and reliable in-position detection solution can be provided by disposing the electrode on the surface of the handle body and determining, by the detection unit based on the change in the voltage of the electrode, whether the finger is in position. This detection solution, using the change in the voltage value as a basis for determination, has a relatively simple circuit, is less susceptible to interference, and exhibits high reliability, thereby avoiding the problem that in-position detection is prone to failure when the finger is relatively moist.

Preferably, the in-position detection module determines, through non-contact sensing of the electrode, whether the finger is in position.

With respect to whether the finger of the user is in position relative to the operating handle, the in-position detection module performs detection through the non-contact sensing of the electrode, to perform in-position detection for a region of the operating handle that requires insulation.

Preferably, the detection unit includes a signal source, a first capacitor, and a first acquisition terminal. The signal source is electrically connected to the electrode and is configured to transmit a first non-direct-current voltage signal to the electrode; the first acquisition terminal is electrically connected to a first terminal of the first capacitor, and a second terminal of the first capacitor is electrically connected to the electrode; the first acquisition terminal acquires a second non-direct-current voltage signal through coupling of the first capacitor; the signal source transmits the first non-direct-current voltage signal to the control module, and the first acquisition terminal transmits the second non-direct-current voltage signal to the control module; and the control module compares a voltage amplitude change of the second non-direct-current voltage signal relative to the first non-direct-current voltage signal to determine whether the finger is in position.

Through the circuit configuration of the detection unit, the non-contact sensing between the electrode and the finger of the user can be detected with relatively simple connections of elements, thereby effectively matching the relatively limited circuit space and reliability requirements of the operating handle.

Preferably, the control module generates a first threshold based on the first non-direct-current voltage signal, and in response to a voltage amplitude of the second non-direct-current voltage signal being less than the first threshold, the control module determines that the finger is in position.

Preferably, the detection unit further includes a first resistor, a first terminal of the first resistor is electrically connected to the signal source, and a second terminal of the first resistor is electrically connected to the electrode.

Circuit protection can be implemented by providing the first resistor to electrically connect the electrode to the signal source.

Preferably, a control button is provided on the handle body, and the control button is correspondingly provided with the in-position detection module.

An insulating pad is overlaid on a surface of the control button, and the electrode of the in-position detection module is disposed on a back side of the insulating pad.

In the case where the insulating pad is provided on the control button on the handle body, the electrode is disposed on the back side of the insulating pad so that the in-position detection function can be fulfilled through the non-contact sensing without affecting the normal operation of the user on the control button. Moreover, the insulating pad can effectively provide insulation and anti-slip functions, thereby improving the operation accuracy of the user for the control button.

Preferably, multiple control buttons are provided on the handle body, and each control button is correspondingly provided with one in-position detection module.

By respectively disposing the in-position detection module on each control button on the handle body, the coverage area of the in-position detection modules on the operating handle can be enlarged, thereby ensuring the accuracy of in-position detection.

Preferably, the in-position detection module determines, through simultaneous contact sensing of an electrode pair, whether the finger is in position.

With respect to whether the finger of the user is in position relative to the operating handle, detection is performed through simultaneous contact sensing of two electrodes of the in-position detection module to determine whether the finger of the user is located within a region in which the electrodes are located.

Moreover, by adjusting the relative positions of the two paired electrodes, the contact position of the finger of the user on the handle body can be constrained so that the finger can be sensed only when the user grips the handle body in a relatively correct manner.

Preferably, the electrode pair includes a third electrode and a fourth electrode, respectively; the detection unit includes a voltage source, a second acquisition terminal, and a third resistor; the voltage source is electrically connected to the third electrode and is configured to apply a first direct-current voltage signal to the third electrode; the fourth electrode is grounded through the third resistor; a first end of the second acquisition terminal is electrically connected to the fourth electrode, and a second end of the second acquisition terminal is electrically connected to the control module; the second acquisition terminal is configured to acquire a second direct-current voltage signal of the fourth electrode and transmit the second direct-current voltage signal to the control module; and the control module compares a voltage value change of the second direct-current voltage signal relative to the first direct-current voltage signal to determine whether the finger is in position.

Through the circuit configuration of the detection unit, contact sensing between the two paired electrodes and the finger of the user can be implemented with relatively simple connections of elements, thereby effectively matching the relatively limited circuit space and reliability requirements of the operating handle.

Preferably, the control module generates a second threshold based on the first direct-current voltage signal, and in response to a voltage value of the second direct-current voltage signal being greater than the second threshold, the control module determines that the finger is in position.

Preferably, the detection unit further includes a second resistor, and the voltage source is electrically connected to the third electrode through the second resistor.

The circuit protection can be implemented by providing the second resistor and the third resistor.

Preferably, the detection unit further includes a filter, and the first end of the second acquisition terminal is electrically connected to the fourth electrode through the filter.

The quality of the signal acquired by the second acquisition terminal can be improved by providing the filter.

Preferably, the second acquisition terminal is a signal converter.

By providing the signal converter, the acquired second direct-current voltage signal can be converted into a signal that is more easily processed by the control module.

Preferably, two electrodes are disposed at an end position of the handle body, and the two electrodes are arranged adjacent to each other on the surface of the handle body.

By arranging the electrode pair of the in-position detection module at the end position of the handle body, effective in-position detection can be performed when the user grips the end of the operating handle to make displacement actions such as flipping, translation, and rotation.

Preferably, the in-position detection module includes multiple electrode pairs alternately arranged on the surface of the handle body.

By alternately arranging the multiple electrode pairs on the surface of the handle body, the coverage area of in-position detection can be enlarged, thereby avoiding the case where the finger of the user contacts only one electrode in the electrode pair and cannot be detected as being in position.

Preferably, multiple in-position detection modules are provided, and electrodes of the multiple in-position detection modules are disposed at different positions of the handle body, respectively.

By respectively disposing the electrodes of the multiple in-position detection modules at different positions of the handle body, the coverage area of in-position detection can be enlarged. By performing the in-position detection at different positions of the handle body, a grip situation of the finger of the user relative to the operating handle can be accurately reflected.

Preferably, the electrode is disposed corresponding to an operable region of the handle body.

By disposing the electrode in the operable region of the handle body, when the user grips a non-operable region of the handle body, the grip cannot be sensed by the electrode, so it is determined that the operating handle is not being held and cannot be activated. Through this structural arrangement, the operation habit of the user can be further constrained, thereby preventing the handle body from being activated under an incorrect grip condition and ensuring that the operating handle can be operated reliably and safely.

An in-position detection method for an operating handle is provided and includes detecting a voltage of the electrode disposed on a surface of the operating handle and determining, based on a change in the detected voltage, whether the finger is in position.

According to the preceding in-position detection method for the operating handle, an accurate and reliable in-position detection solution can be provided by detecting the change in the voltage of the electrode disposed on the surface of the handle body and using the change in the voltage as a basis for determining whether the finger is in position. This detection solution, using the change in the voltage as the basis for determination, has a relatively simple circuit, is less susceptible to interference, and exhibits high reliability, thereby avoiding the problem that the in-position detection is prone to failure when the finger is relatively moist.

Preferably, multiple electrodes are distributed on the surface of the operating handle; the step in which the voltage of the electrode disposed on the surface of the operating handle is detected and it is determined based on the change in the detected voltage whether the finger is in position includes the following.

Voltages of the multiple electrodes disposed on the surface of the operating handle are simultaneously detected, and in response to a voltage of at least one electrode being lower than a threshold, it is determined that the finger is in position.

By measuring the multiple electrodes and using the voltage of one electrode being lower than the threshold as a basis for determining that the finger is in position, the detection range of the in-position detection method on the operating handle can be expanded, thereby avoiding a detection error caused when the finger of the user is located in a region of the operating handle without an electrode.

The advantageous technical effects of the present application are as follows.

In the present application, the electrode is disposed on the surface of the handle body, and the detection unit determines, based on the change in the voltage of the electrode, whether the finger is in position. This detection solution, using the change in the voltage as the basis for determination, has a relatively simple circuit, is less susceptible to interference, and exhibits high reliability, thereby avoiding the problem that the in-position detection is prone to failure when the finger is relatively moist. Moreover, based on the contact manner between the finger and the operating handle and considering the small volume of the operating handle, a correspondingly adapted finger in-position detection circuit is provided, thereby performing accurate detection of the grip of the user on the operating handle.

### BRIEF DESCRIPTION OF DRAWINGS

FIG. 1 is a structural view of an operating handle according to a preferred embodiment of the present application.
FIG. 2 is a diagram illustrating the layout relationship between a handle body and electrodes according to a preferred embodiment of the present application.
FIG. 3 is a schematic diagram of a control system of an operating handle according to a preferred embodiment of the present application.
FIG. 4 is a circuit diagram of a first electrode according to a preferred embodiment of the present application.
FIG. 5 is a schematic diagram of in-position detection performed by a first electrode according to a preferred embodiment of the present application.
FIG. 6 is a circuit diagram of a third electrode and a fourth electrode according to a preferred embodiment of the present application.
FIG. 7 is a schematic diagram of in-position detection performed by a third electrode and a fourth electrode according to a preferred embodiment of the present application.

### Reference list

- 1: handle body
- 11: control button
- 111: insulating pad
- 20: first electrode
- 30: second electrode
- 40: third electrode
- 50: fourth electrode

### DETAILED DESCRIPTION

The present application is further illustrated below with reference to embodiments. However, the present application is not limited to the scope of the described embodiments.

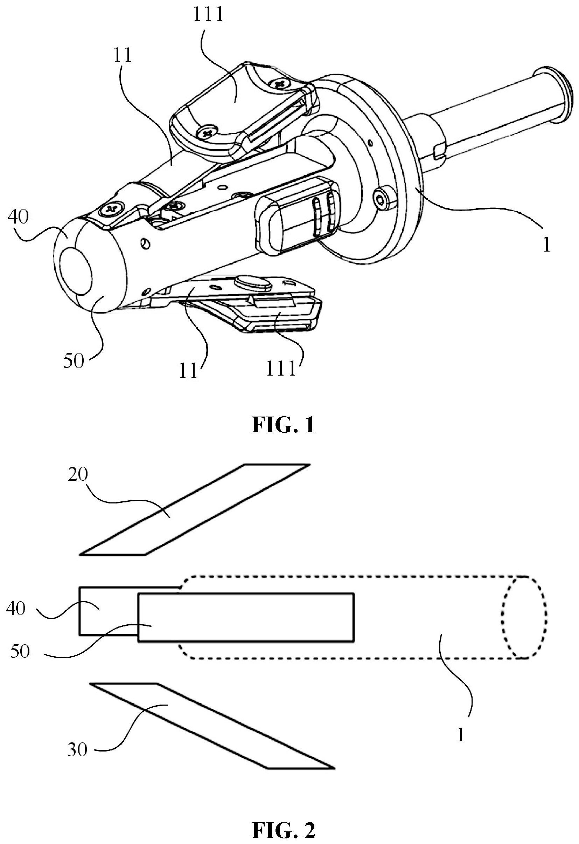

As shown in FIGS. 1 to 3, the present application provides an operating handle. The operating handle includes a handle body 1. The operating handle is gripped by a surgeon to control a distal instrument to synchronously move. To prevent the operating handle from slipping out of the hand of the surgeon and causing an inadvertent contact, the present application further provides an in-position detection module and a control module for detecting whether the finger of the surgeon is in position.

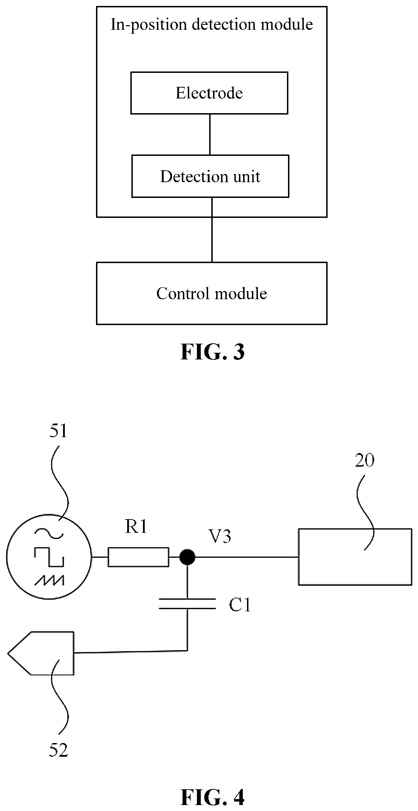

As shown in FIG. 3, finger in-position detection of the operating handle includes the in-position detection module and the control module. The in-position detection module specifically includes an electrode and a detection unit. By disposing the electrode on the surface of the handle body 1, the detection unit is electrically connected to the electrode to acquire the voltage of the electrode through the detection unit and transmit the value of the voltage to the control module. The control module determines, based on a change in the voltage, whether the operating handle is being held. If the control module determines that the finger is in position, the entire system enters a surgeon control mode, enabling the surgeon to control the distal instrument (such as an operating arm) to move through the operating handle. If the control module determines that the finger has left, the entire system enters a locking mode, in which any operation on the operating handle cannot control the distal instrument to move, thereby promptly locking the distal instrument when the hand of the surgeon is withdrawn from the operating handle or when the operating handle slips from the hand, and avoiding surgical risks. The solution, which is based on the change in the voltage of the electrode under the action of the finger, can effectively distinguish whether the electrode is acted upon by the finger or by other objects. Compared with the existing solution in which infrared distance sensors are disposed, or image recognition is performed via video capturing, this solution can effectively avoid misjudgment.

Specifically, as shown in FIGS. 1 and 2, considering the relatively small volume of the operating handle and based on the actual contact manner of fingers with the operating handle, four electrode plates are arranged on the handle body 1 to correspond to two operating modes of the operating handle, thereby improving the reliability of finger in-position detection.

One operating mode of the operating handle is to pinch two control buttons 11 symmetrically disposed on the handle body 1 with fingers. The primary contact and force-receiving positions are on the surfaces of insulating pads 111 of the two control buttons 11. By pressing the two control buttons 11 with fingers, the distal instrument (such as scissors) is controlled to be closed; conversely, by releasing the two control buttons with fingers, the distal instrument (such as scissors) is controlled to be opened. To perform in-position detection, the electrode of the in-position detection module is provided on a back side of each insulating pad 111. That is, a first electrode 20 and a second electrode 30 are disposed on the back sides of upper and lower insulating pads 111 in FIG. 1, respectively. The conditions of fingers on the corresponding control buttons 11 are detected through the electrodes of the two in-position detection modules, to accurately determine whether the fingers are in position.

The other operating mode of the operating handle is to pinch the end position of the handle body 1 with fingers, to control the handle body 1 to rotate, thereby controlling the distal instrument to synchronously rotate. A third electrode 40 and a fourth electrode 50 are provided at the end of the handle body 1, and whether the fingers are in position is determined by detecting the fingers.

Regarding the first operation solution, since the control buttons 11 of the handle body 1 are provided with the insulating pads 111, respectively, the fingers cannot directly contact the electrodes. Therefore, the first electrode 20 and the second electrode 30 determine whether the fingers are in position through non-contact sensing. In this embodiment, a relatively preferred circuit layout of the in-position detection module is provided. Specifically, as shown in FIG. 4, an in-position detection module corresponding to the first electrode 20 on the handle body 1 is used as an example. The detection unit of the in-position detection module includes a signal source 51, a first acquisition terminal 52, and a first capacitor C1. The signal source 51 is electrically connected to the first electrode 20 through a first resistor R1 and is configured to transmit a first non-direct-current voltage signal to the first electrode 20. The first acquisition terminal 52 is electrically connected to a first terminal of the first capacitor C1, and a second terminal of the first capacitor C1 is electrically connected to the first electrode 20 so that the first acquisition terminal 52 can acquire a second non-direct-current voltage signal by coupling the first capacitor C1 to the circuit. The signal source 51 transmits the first non-direct-current voltage signal to the control module, and the first acquisition terminal 52 transmits the second non-direct-current voltage signal to the control module. By comparing the voltage amplitude difference between the first non-direct-current voltage signal and the second non-direct-current voltage signal, the control module can determine the position of a finger relative to the first electrode 20, that is, determine whether the finger is in position. If the voltage amplitude difference between the first non-direct-current voltage signal and the second non-direct-current voltage signal is greater than a first threshold, the control module determines that the finger is in position, and the entire system enters the surgeon control mode. For example, in this embodiment, the signal source 51 outputs a rectangular wave having a voltage amplitude of 3.3 V and a frequency within a range of 100 kHz to 1000 kHz. In this case, an optional range of the first threshold is from 300 mV to 500 mV.

In this embodiment, the operating handle further includes a microcontroller unit and a power management unit. The microcontroller unit (MCU) is configured to generate excitation signals for the electrodes and acquire the signals, to determine the in-position logic based on the acquired signals and output a result. The power management unit is configured to supply power to various functional units. The signal source 51 and the first acquisition terminal 52 of the in-position detection module of the operating handle, and the entire control module can all be implemented by the microcontroller unit (MCU) of the operating handle. The first threshold used to determine whether the finger is in position is also stored in the microcontroller unit so that in-position detection can be performed by controlling the output and acquisition of signals. For example, the microcontroller unit outputs, through a digital-to-analog converter (DAC), a pulse signal, a rectangular wave signal, or a sine wave signal, which has a continuous amplitude of VCC, as the first non-direct-current voltage signal, and the voltage amplitude VCC may be 5 V or 3.3 V. In other embodiments, other integrated circuits (ICs) may also be used for in-position detection.

When the finger is away from the first electrode 20, as shown in FIG. 4, the first non-direct-current voltage signal from the signal source is essentially intact and coupled to the first acquisition terminal 52 through the first capacitor C1 at V3 after passing through the first resistor R1. The second non-direct-current voltage signal acquired by the first acquisition terminal 52 has an amplitude substantially consistent with that of the first non-direct-current voltage signal from the signal source 51. That is, the voltage amplitude difference between the first non-direct-current voltage signal and the second non-direct-current voltage signal is less than the first threshold. In this case, the control module of the microcontroller unit determines that the finger is not in position, and the entire system enters the locking mode, in which any operation on the handle body 1 cannot control the distal instrument.

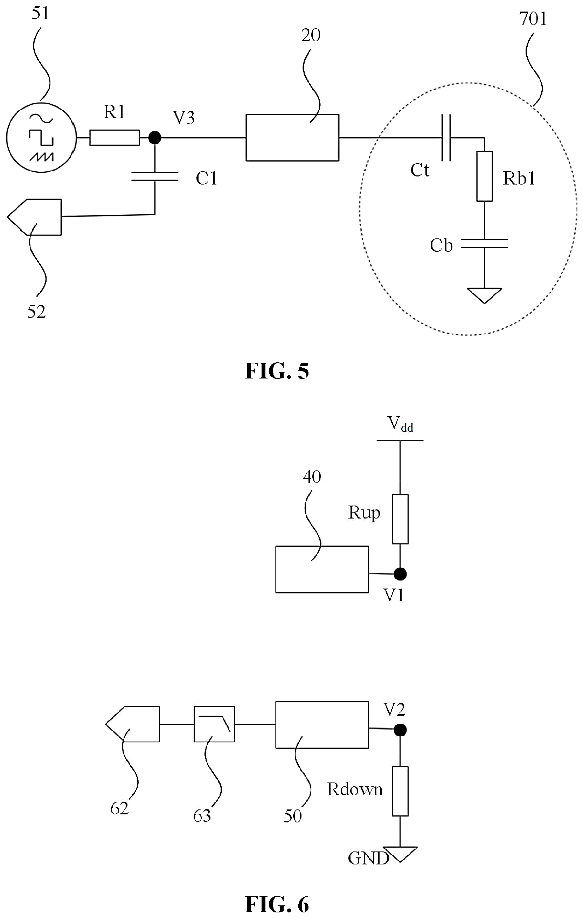

When the finger is close to the first electrode 20, as shown in FIG. 5, the capacitive characteristics of the finger become apparent, which is equivalent to connecting a first finger equivalent circuit 701 to the first electrode 20, thereby forming a second loop between the first electrode 20 and the ground terminal. In this case, both the amplitude and phase of the second non-direct-current voltage signal acquired by the first acquisition terminal 52 change, and the amplitude of the second non-direct-current voltage signal coupled to the first acquisition terminal 52 through the first capacitor C1 decreases. The control module determines whether the amplitude change between the first non-direct-current voltage signal and the second non-direct-current voltage signal is greater than the first threshold, to determine whether the finger of the surgeon is in position. If it is determined that the finger is in position, the entire system enters the surgeon control mode. The first finger equivalent circuit 701 is formed by a complex coupling of resistors and capacitors with different sizes, to represent the impedance characteristics of the human body via the finger. Specifically, the impedance of the human finger and the impedance of the human body exhibit different characteristics under different electrical excitation. An equivalent impedance model is obtained by comparing changes in the amplitude and phase between the output and input of an impedance measurement instrument in the frequency domain after an excitation signal passes through the human body and is measured by the impedance measurement instrument.

The schematic diagram of in-position detection of the second electrode 30 is the same as that of the first electrode 20. When a finger approaches the second electrode 30, both the amplitude and phase of the second non-direct-current voltage signal obtained by the first acquisition terminal 52 corresponding to the second electrode 30 also change. This can allow the control module to determine whether the amplitude change between the first non-direct-current voltage signal and the second non-direct-current voltage signal is greater than the first threshold to determine whether the finger of the surgeon is in position relative to the second electrode 30.

This circuit configuration for implementing non-contact sensing can detect the non-contact sensing between the electrode and the finger of the user with relatively simple combinations and connections of elements, thereby effectively matching the relatively limited circuit space and reliability requirements of the operating handle.

Regarding the second operation solution, the third electrode 40 and the fourth electrode 50 that are arranged in a pair are disposed at the end position of the handle body 1. By simultaneous contact of fingers with both the third electrode 40 and the fourth electrode 50, the fingers can be sensed, thereby serving as the basis for determining whether the fingers are in position. In this embodiment, a relatively preferred circuit layout of an in-position detection module is provided. Specifically, as shown in FIG. 6, the detection unit corresponding to the third electrode 40 and the fourth electrode 50 includes a voltage source Vdd and a second acquisition terminal 62. The voltage source Vdd is electrically connected to the first electrode 20 through a second resistor Rup and is configured to apply a first direct-current voltage signal to the first electrode 20. The second electrode 30 is grounded through a third resistor Rdown. A first end of the second acquisition terminal 62 is electrically connected to the fourth electrode 50, and a second end of the second acquisition terminal 62 is electrically connected to the control module. The second acquisition terminal 62 is configured to acquire a second direct-current voltage signal of the fourth electrode 50 and transmit the second direct-current voltage signal to the control module. The control module determines, by comparing the voltage value difference between the second direct-current voltage signal and a ground voltage, whether the fingers are in position. Specifically, if the voltage value difference between the second direct-current voltage signal and the ground voltage is greater than a second threshold, the control module determines that the fingers are in position, and the entire system enters the surgeon control mode.

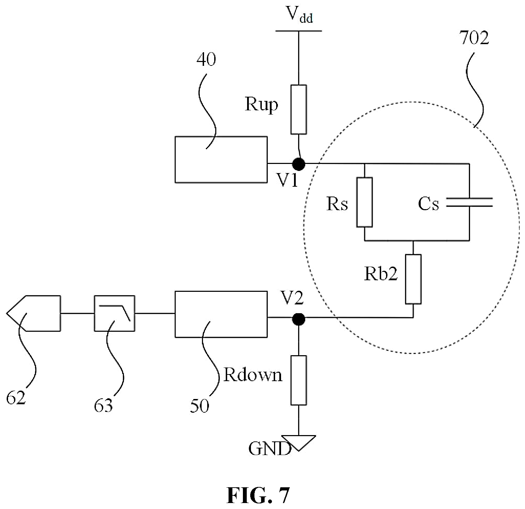

Specifically, as shown in FIG. 6, when the fingers do not simultaneously contact the third electrode 40 and the fourth electrode 50, the connection between the third electrode 40 and the fourth electrode 50 is equivalent to a complete open circuit. In this case, the voltage of the third electrode 40 is pulled up to Vdd by the second resistor Rup, which is approximately 3.3 V in this embodiment, while the voltage of the fourth electrode 50 is pulled down to GND by the third resistor Rdown. As shown in FIG. 7, when the fingers simultaneously pinch the third electrode 40 and the fourth electrode 50, due to an equivalent impedance model of the human body, the connection between the third electrode 40 and the fourth electrode 50 is equivalent to being connected by a resistive-capacitive device, which is equivalent to connecting a second finger equivalent circuit 702 between the third electrode 40 and the fourth electrode 50. At this time, the voltage of the third electrode 40 and the voltage of the fourth electrode 50 change: the voltage at the third electrode 40 decreases, while the voltage at the fourth electrode 50 increases. The current second direct-current voltage signal of the fourth electrode 50 is acquired through the second acquisition terminal 62, and the control module determines, by comparing the second direct-current voltage signal with a set threshold, whether the fingers of the surgeon are in position. The second finger equivalent circuit 702 is formed by a complex coupling of resistors and capacitors with different sizes, to represent the impedance characteristics of the human body via the fingers. Specifically, the impedance of a human finger and the impedance of the human body exhibit different characteristics under different electrical excitation. An equivalent impedance model is obtained by comparing changes in the amplitude and phase between the output and input of an impedance measurement instrument in the frequency domain after an excitation signal passes through the human body and is measured by the impedance measurement instrument.

The second resistor Rup and the third resistor Rdown have the same resistance value, which is in the range from 1 MΩ to 10 MΩ. Since the impedance value formed by the fingers is on the order of kilohms, when the fingers simultaneously contact the third electrode 40 and the fourth electrode 50, the range of voltage values obtained at V1 and V2 is around Vdd/2. Therefore, by setting the second threshold to a value less than Vdd/2, in a case where the second direct-current voltage signal is greater than the second threshold, the control module determines that the fingers are in position, thereby enabling the entire system to enter the surgeon control mode. Specifically, in this embodiment, the voltage value Vdd may be 3.3 V or 5 V; in this case, an optional range of the second threshold is from 500 mV to 1400 mV.

This circuit configuration for contact sensing can detect the contact sensing between the two paired electrodes and the fingers of the user with relatively simple connections of elements, thereby effectively matching the relatively limited circuit space and reliability requirements of the operating handle.

Specifically, as shown in FIG. 6, the detection unit further includes a filter 63, specifically a low-pass filter 63. The first end of the second acquisition terminal 62 is electrically connected to the fourth electrode 50 through the low-pass filter 63. The quality of the signal acquired by the second acquisition terminal 62 can be improved by providing the low-pass filter 63. Further, in this embodiment, the second acquisition terminal 62 is a signal converter, specifically an analog-to-digital converter, to convert an acquired analog signal into a digital signal, thereby facilitating processing by the microcontroller unit (MCU).

With respect to the specific arrangement positions of the third electrode 40 and the fourth electrode 50 on the handle body 1, as shown in FIG. 1, the two electrodes are arranged adjacent to each other on the surface of the handle body 1 so that effective in-position detection can be performed when the user grips the end of the operating handle to make the displacement actions such as flipping, translation, and rotation.

In this embodiment, the third electrode 40 and the fourth electrode 50 are arranged at the end position of the handle body 1, to perform the in-position detection when the user grips the end of the operating handle. To perform the in-position detection, the fingers must simultaneously contact the third electrode 40 and the fourth electrode 50 to form a loop. Therefore, a loop cannot be formed in a case where only one of the third electrode 40 or the fourth electrode 50 is in contact with a finger, which leads to a determination in the in-position detection that the fingers have left. To solve this problem, in this embodiment, the third electrode 40 and the fourth electrode 50 are arranged adjacent to each other at the end of the handle body 1, to reduce the probability that the fingers contact only one of the electrodes. In other embodiments, multiple electrode pairs may be further provided, and the multiple electrode pairs may be alternately arranged on the surface of the handle body 1, to further reduce the occurrence of the case where a loop cannot be formed due to the contact of the finger with only one electrode.

In addition, in other embodiments, the multiple electrode pairs may be further arranged at different positions of the handle body 1, respectively, to further enlarge the coverage area of the in-position detection on the handle body 1. By performing the in-position detection at different positions of the handle body 1, the grip condition of the fingers of the user relative to the operating handle can be accurately reflected. Certainly, these electrodes should be arranged within an operable region of the handle body 1 so that a grip of the user at a non-operable region of the handle body 1 cannot be sensed by the electrodes. In this manner, the microcontroller unit of the operating handle determines that the fingers are in position, and the operating handle cannot be activated. Through this arrangement of the electrodes, the operation habit of the user can be further constrained, thereby preventing the handle body 1 from being activated under an incorrect grip condition and ensuring that the operating handle can be operated reliably and safely.

In addition, the present application further provides an in-position detection method for an operating handle. The method specifically includes the following: detecting the voltage of an electrode disposed on the surface of the operating handle and determining, based on a change in the detected voltage, whether the finger is in position. In this in-position detection solution, the change in the voltage of the electrode is used as the basis for determining whether the finger is in position. In this manner, an accurate and reliable in-position detection solution can be provided. This detection solution, using the change in the voltage as the basis for determination, has a relatively simple circuit, is less susceptible to interference, and exhibits high reliability, thereby avoiding the problem that the in-position detection is prone to failure when the finger is relatively moist.

Further, since the electrodes are arranged at multiple positions of the operating handle, voltages of these electrodes can be detected simultaneously; if the voltage of one electrode is lower than a threshold, it is determined that the finger is in position.

The specific implementation steps of the in-position detection are as follows.
1. After the entire control system is powered on, the microcontroller unit applies the first non-direct-current voltage signal to the first electrode 20 and the second electrode 30 and simultaneously applies the first direct-current voltage signal to the third electrode 40 to pull up the voltage of the third electrode 40 to Vdd, while the fourth electrode 50 is grounded to pull down the voltage of the fourth electrode 50 to GND. The first acquisition terminal 52 and the second acquisition terminal 62 continuously acquire the second non-direct-current voltage signal and the second direct-current voltage signal. Moreover, the system generates the first threshold Vth1 based on the voltage amplitude of the first non-direct-current voltage signal and generates the second threshold Vth3 based on the voltage value of the first direct-current voltage signal.
2. When the fingers of the surgeon are placed on the first electrode 20 and the second electrode 30 to pinch the handle body 1, it can be seen from the first finger equivalent circuit 701 in FIG. 5, a series loop of capacitors and resistors is formed between the fingers and the first electrode 20 or the second electrode 30, so the amplitude of the voltage signal at V3 in the circuit decreases, causing a change in the voltage amplitude of the second non-direct-current voltage signal acquired by the first acquisition terminal 52, and the voltage amplitude becomes less than the first threshold Vth1. Accordingly, the control module of the microcontroller unit determines that the fingers are in position, and the system enters the surgeon control mode.
3. Conversely, when the fingers leave the first electrode 20 and the second electrode 30, the voltage amplitude at V3 increases, causing a change in the voltage amplitude of the second non-direct-current voltage signal acquired by the first acquisition terminal 52, and the voltage amplitude becomes greater than the first threshold Vth1. The control module of the microcontroller unit determines that the fingers have left, and the system enters the locking mode.
4. When the fingers of the surgeon are placed on the third electrode 40 and the fourth electrode 50 to rotate the operating handle, it can be seen from the second finger equivalent circuit 702 in FIG. 6 that a parallel loop of capacitors and resistors is formed between the fingers, the third electrode 40, and the fourth electrode 50. The second resistor Rup and the third resistor Rdown have the same resistance value, which is in the range from 1 MΩ to 10 MΩ. Since the impedance value formed by the fingers is on the order of kilohms, the range of the voltage values at V1 and V2 in the circuit is around Vdd/2. The voltage value of the second direct-current voltage signal acquired by the second acquisition terminal 62 changes and is greater than the second threshold Vth3. Accordingly, the control module of the microcontroller unit determines that the fingers are in position, and the system enters the surgeon control mode.
5. Conversely, when the fingers leave the third electrode 40 and the fourth electrode 50, the voltage value at V2 becomes GND, causing the voltage value of the second direct-current voltage signal acquired by the second acquisition terminal 62 to be less than the second threshold Vth3. The control module of the microcontroller unit determines that the fingers have left, and the system enters the locking mode.

The non-contact in-position detection solution for the first electrode 20 and the second electrode 30 and the contact in-position detection solution for the third electrode 40 and the fourth electrode 50 each can independently fulfill the function of determining whether the fingers are in position, or may be used in combination according to the design requirements and application scenarios of the operating handle.

In other embodiments, if the second acquisition terminal 62 is electrically connected to the third electrode 40 to acquire the change in the voltage value at V1, the second threshold Vth4 is set based on the voltage value Vdd/2. When the voltage value of the second direct-current voltage signal acquired by the second acquisition terminal 62 is less than the second threshold Vth4, it is determined that the fingers are in position, and the system enters the surgeon control mode. When the voltage value of the second direct-current voltage signal acquired by the second acquisition terminal 62 is greater than the second threshold Vth4, it is determined that the fingers have left, and the system enters the locking mode.

Although the specific embodiments of the present application have been described above, those skilled in the art should understand that these are illustrative. The scope of protection of the present application is defined by the appended claims. Without departing from the principles and essence of the present application, those skilled in the art can make various changes and modifications to these embodiments, and these changes and modifications shall fall within the scope of protection of the present application.

## Claims

1. An operating handle, comprising a handle body, an in-position detection module, and a control module, wherein the in-position detection module comprises:
an electrode disposed on a surface of the handle body; and
a detection unit, which is electrically connected to the electrode and is communicatively connected to the control module, wherein the detection unit is configured to acquire a voltage of the electrode; and
wherein the control module is configured to determine, based on a change in the voltage, whether a finger is in position.

2. The operating handle according to claim 1, wherein the in-position detection module is configured to determine, through non-contact sensing of the electrode, whether the finger is in position.

3. The operating handle according to claim 1 or 2, wherein the detection unit comprises a signal source, a first capacitor, and a first acquisition terminal; the signal source is electrically connected to the electrode and is configured to transmit a first non-direct-current voltage signal to the electrode; the first acquisition terminal is electrically connected to a first terminal of the first capacitor, and a second terminal of the first capacitor is electrically connected to the electrode; the first acquisition terminal is configured to acquire a second non-direct-current voltage signal through coupling of the first capacitor; the signal source is configured to transmit the first non-direct-current voltage signal to the control module, and the first acquisition terminal is configured to transmit the second non-direct-current voltage signal to the control module; and the control module is configured to compare a voltage amplitude change of the second non-direct-current voltage signal relative to the first non-direct-current voltage signal to determine whether the finger is in position.

4. The operating handle according to claim 3, wherein the control module is configured to generate a first threshold based on the first non-direct-current voltage signal, and in response to a voltage amplitude of the second non-direct-current voltage signal being less than the first threshold, the control module is configured to determine that the finger is in position; and/or
the detection unit further comprises a first resistor, a first terminal of the first resistor is electrically connected to the signal source, and a second terminal of the first resistor is electrically connected to the electrode.

5. The operating handle according to any one of claims 1 to 4, wherein the handle body is provided with a control button, and the in-position detection module is correspondingly provided on the control button; and
an insulating pad is overlaid on a surface of the control button, and the electrode of the in-position detection module is disposed on a back side of the insulating pad.

6. The operating handle according to any one of claims 1 to 4, wherein the handle body is provided with a plurality of control buttons, and each control button of the plurality of control buttons is correspondingly provided with one in-position detection module.

7. The operating handle according to any one of claims 1 to 6, wherein the in-position detection module is configured to determine, through simultaneous contact sensing of an electrode pair, whether the finger is in position.

8. The operating handle according to claim 7, wherein the electrode pair comprises a third electrode and a fourth electrode; the detection unit comprises a voltage source, a second acquisition terminal, and a third resistor; the voltage source is electrically connected to the third electrode and is configured to apply a first direct-current voltage signal to the third electrode; the fourth electrode is grounded through the third resistor; a first end of the second acquisition terminal is electrically connected to the fourth electrode, and a second end of the second acquisition terminal is electrically connected to the control module; the second acquisition terminal is configured to acquire a second direct-current voltage signal of the fourth electrode and transmit the second direct-current voltage signal to the control module; and the control module is configured to compare a voltage value change of the second direct-current voltage signal relative to the first direct-current voltage signal to determine whether the finger is in position.

9. The operating handle according to claim 8, wherein the control module is configured to generate a second threshold based on the first direct-current voltage signal, and in response to a voltage value of the second direct-current voltage signal being greater than the second threshold, the control module is configured to determine that the finger is in position; and/or
the detection unit further comprises a second resistor, and the voltage source is electrically connected to the third electrode through the second resistor; and/or
the detection unit further comprises a filter, and the first end of the second acquisition terminal is electrically connected to the fourth electrode through the filter; and/or
the second acquisition terminal is a signal converter.

10. The operating handle according to any one of claims 1 to 9, wherein two electrodes are disposed at an end position of the handle body, and the two electrodes are arranged adjacent to each other on the surface of the handle body.

11. The operating handle according to claim 10, wherein the in-position detection module comprises a plurality of electrode pairs, and the plurality of electrode pairs are alternately arranged on the surface of the handle body.

12. The operating handle according to any one of claims 1 to 11, wherein a plurality of in-position detection modules are provided, and electrodes of the plurality of in-position detection modules are disposed at different positions of the handle body, respectively; and/or the electrode is disposed corresponding to an operable region of the handle body.

13. An in-position detection method for an operating handle, wherein the operating handle is the operating handle according to any one of claims 1 to 12, and the method comprises: detecting a voltage of the electrode disposed on a surface of the operating handle and determining, based on a change in the detected voltage, whether the finger is in position.

14. The in-position detection method for the operating handle according to claim 13, wherein a plurality of electrodes are distributed on the surface of the operating handle; detecting the voltage of the electrode disposed on the surface of the operating handle and determining, based on the change in the detected voltage, whether the finger is in position comprises:
simultaneously detecting voltages of the plurality of electrodes disposed on the surface of the operating handle, and in response to a voltage of at least one electrode of the plurality of electrodes being lower than a threshold, determining that the finger is in position.
